# EUROPEAN PATENT APPLICATION

(11) **EP 2 591 667 A1**
(43) Date of publication of application: **15.05.2013**
(21) Application number: 12195780.7
(22) Date of filing: 11.11.2008
(51) Int. Cl.: A01H 5/10, A21D 2/18, C08B 30/04, A23L 1/16

(54) **High amylose durum wheat grains, meal and starch**

(62) Divisional of application: 08425721.1
(71) Applicant: Barilla G. e R. Fratelli S.p.A., 43100 Parma (IT)
(72) Inventor: Silvestri, Marco, 41100 Modena (IT); Ranieri, Roberto, 43052 Colorno (Parma) (IT); Recchia, Giovanna, 43100 Parma (IT); Rossi, Stefano, 43022 Montechiarugolo (Parma) (IT); Arlotti, Guido, 43100 Parma (IT); Lafiandra, Domenico, 01027 Montefiascone (Viterbo) (IT); Deambrogio, Enzo, 44042 Cento (Ferrara) (IT)
(74) Representative: Ferreccio, Rinaldo

(57) **Abstract**

The invention refers to grain of durum wheat, selected for the characteristic of not being capable of expressing the *Sgp-1* gene, containing a durum wheat starch with an apparent amylose content of about 40% or more, by weight on the weight of the starch. Furthermore, the invention refers to a durum wheat meal obtained from the endosperm of caryopses of durum wheat, selected for the characteristic of not being capable of expressing the *Sgp-1* gene, containing a durum wheat starch with an apparent amylose content of about 40% or more, by weight on the weight of the starch. Furthermore, the present invention refers to a durum wheat starch obtained from the endosperm of caryopses of durum wheat, selected for the characteristic of not being capable of expressing the *Sgp-1* gene, having an apparent amylose content of about 40% or more by weight on the weight of the starch. Finally, the present invention refers to the use of the grain, meal and starch of the invention for the production of food products, in particular pastas, bread, biscuits and cakes, having prebiotic effect and a reduced glycemic index.

## Description

### Field of application

The present invention refers to the technical field of the food industry, in particular to a durum wheat grain or a meal (flour or semolina), containing high amylose starch and to the high amylose starch.

Furthermore, the present invention refers to the use of the high amylose durum wheat grain, meal and starch of the invention in the production of food products having prebiotic effect and a reduced glycemic index.

Additionally, the present invention refers to the food products having high amylose content and containing the grains and/or meal and/or starch of the invention, in particular pastas having prebiotic effect and reduced glycemic index.

### State of the art

As provided for by the specific Italian legislation (law 580/67 and subsequent amendment by Presidential Decree n°117, art. 6 of February 9, 2001), only pasta produced by the exclusive use of durum wheat meal (semolina), is capable of guaranteeing high quality performance (firmness upon cooking) in line with traditional criteria ("al dente" firmness).

As known, the wheat caryopsis mainly consists of starch. Starch is a polymer consisting of repeated D-Glucose units which, depending on the type of structure and bonds present, may be classified as amylose or amylopectin. These two polymers are concentrated in the form of granules which form the prevailing part of the endosperm cells. Durum wheat starch contains about 20-25% of amylose and 75-80% of amylopectin.

Amylose consists of long chains of α-D-glucopyranoside units joined by α(1→4) bonds. Such chain also comprises a modest amount of branching. An amylose molecule has a degree of polymerisation ranging between 10³ and 10⁴ units (Ball *et al.,* 1998) and it has an average molecular weight of 2 x 10⁶ (Coultate, 2001).

Amylopectin, instead, is a much larger molecule than amylose, with a degree of polymerisation comprised between 10⁵ and 10⁷ glucose units joined through α(1→4) bonds. Differently than amylose, 4-5% of the glucose units are bound through α(1→6) glucosidic bonds, which form branching points (Morell *et al.,* 1995). The length of the branches is generally of 20-25 units.

Due to the presence of α(1→4) bonds, amylose tends to acquire a α-helix configuration, stabilised by the possibility for the oxydryl groups distributed along its chain to form many hydrogen bonds between them, thus creating, at its centre, an apolar cavity. Such cavity, due to its hydrophobicity, has a tendency to form inclusion complexes with the aliphatic chains of the fatty acids (*LAM, lipid-complexed amylose*)*.*

The chemical and physical characteristics of starch, especially when it is exposed to humidity or submerged in water, are not so much due to the total amount of starch present but in particular to the relative amounts of amylose and amylopectin, which determine a specific viscoelastic behaviour and functionality.

Amylose, in particular, if present in concentrations above 1.5 %, forms cohesive gels responsible for starch retrogradation, a phenomenon that occurs in food processing. A high concentration of this molecule gives starch a low hydration level and a low swelling capacity of the starch granule. In fact, lipid-amylose complexes are insoluble in water. The insoluble complex, located at the surface of the starch granules, slows its water absorption, preventing starch gelatinization (Iturriaga *et al.,* 2006; Raphaelides *et al.,* 2008).

On the contrary, a high concentration of amylopectin allows the formation of gels but delays or prevents retrogradation. In fact, it confers high hydration and swelling capacity on the starch molecule (Yasui *et al.,* 1996). Amylopectin gelatinization is an irreversible process during which the swelling of starch granules can be observed, under the action of water, which diffuses towards the inside and solubilizes the amylopectin crystalline regions; the phenomenon occurs only above the glass transition temperature of the starch granules (50 - 70 °C, depending on the starch structure and composition, and the plant source) and in the presence of an excess of water.

The wheat caryopsis is made up 70% in weight by starch, and so it follows that controlling the amylose to amylopectin ratio represents a key factor in the modulation of the functional characteristics of starch and for the end product quality (Rahman et *al.,* 2000).

For example, in the case of pasta, it is known that such ratio has an impact on the firmness of the pasta upon cooking in boiling water. Another characteristic affected by such ratio, in the case of pasta cooked in boiling water, is the variation in the thickness of the pasta (Sharma et *al.,* 2002),

In fact, it has been demonstrated for wheat starch that there is a positive correlation between the quality of the pasta and the amylose content (Sharma et *al.,* 2002).

Furthermore, it is recognized that the texture of cooked pasta is the most important parameter in the evaluation of the overall quality of pasta. Ideally, cooked pasta should be firm and resilient, with no surface stickiness and the cooking loss should be minimal.

It is also known that high amylose starch with a high amylose content with respect to the amylopectin content is associated to a reduction in the glycemic index (GI), as a consequence of a lower susceptibility (retrogradation) to the action of the digestive enzymes (resistant starch). The intake of a low GI food product is, in turn, correlated to health-promoting effects, such as, for example, a reduction in the risk of chronic diseases such as diabetes, certain types of cancer and coronary heart diseases (Hu et *al.,* 2004; Foster-Powell et *al.,* 2002; King et *al.,* 2008; Topping, 2007).

To produce high amylose durum wheat pasta, thus capable of maintaining the aforementioned parameters at an ideal and functional level, there arises the need to provide durum wheat grain and/or meal containing starch having a high content of amylose compared to amylopectin.

The US patent application No. 2006/0240099 (Carbone et al.*)* describes a process for preparing high amylose starch having an apparent density lower than 0.4 g/ml and average particle size of 100 micrometres. The process involves the steps of forming a suspension comprising a high amylose starch and water, and subjecting the suspension to steam jet cooking at 125-135 °C so as to obtain a pregelatinized high amylose starch paste. Finally, the pregelatinised starch paste must be cooked and dried on a drum drier at a pressure of between 2 and 10 bar, so as to obtain a pregelatinized high amylose starch.

In spite of the high amylose yield obtained through such a process, there is clearly the need of having a raw material, in this specific case high amylose starch extracted from plants such as corn, potatoes and tapioca, that must be subjected to a series of technological treatments aimed at concentrating and purifying such starch fraction. The amylose thus obtained is subsequently useable as a component for preparing capsules and/ or coating films for coating and protecting drugs, food for human and animal consumption and seeds.

The chemical modification of starch is commonly used in the field for lowering the gelatinization temperature and the rate of retrogradation. However, the chemical processes require a considerable input of energy and they involve the need to recover the waste-products of unreacted reagents and salts from the waste water. Thus, these processes are expensive and inadequately sustainable from an environmental point of view.

Thus, it would be desirable to be able to control starch biosynthesis in durum wheat so that it had characteristics that directly conferred on the products obtained therefrom the respective improved organoleptic and nutritional characteristics. In this manner, use of additional, costly, generally poorly effective and environmentally inadequately sustainable transformation processes would not be necessary.

There are two main approaches for inserting suitable genetic characters into a plant species such as for example wheat: manipulation through genetic engineering, and conventional breeding methods. The main genetic engineering technique applicable on wheat is particle bombardment, though it is possible to obtain transgenic plants also through *Agrobacterium* -mediated DNA transfer (Jones, 2005)

In the US patent No. 7,001,939, Yamamori describes common wheat starch obtained from the endosperm of a seed of natural mutants of wheat lacking the *starch granule protein-1* (SGP-1). The starch of such wheat has an apparent amylose content of about 35% or more.

In US 6,635,756 Jobling et al. describes a starch obtained from plants modified by manipulating the activity of a combination of enzymes having starch synthesis activities, in particular the starch synthase enzymes II (SSII) and III (SSIII).

Another approach is indicated by Jane *et al.* which, in the US 7,009,092 patent, describes the genetic modulation of corn starch wherein the characteristics of corn starch were modified expressing in a corn plant a non-corn starch branching enzyme. In particular, the barley starch branching enzyme SBE IIa *(starch branching enzyme) transgene* was used. Such modification affected the gelatinization temperature and the rate of retrogradation of the corn starch produced.

Regina *et al.* (2006) describe an experiment with wheat wherein, the expression of enzymes involved in starch branching *(starch branching enzyme)* SBE IIa and SBE IIb was suppressed through genetic engineering. This led to a starch having an amylose content greater than 70%. When the wheat thus modified was administered to rats, it was observed that it contributed to the improvement of certain aspects of intestinal function such as Short Chain Fatty Acids (SCFA).

However, it should be observed that, though effective, genetic modification to obtain the desired amylose to amylopectin ratio has the drawback that the product obtained therefrom is perceived by the average consumer as being artificial and potentially dangerous. In fact, commercial use of transgenic technologies in the food industry remains, as of date, dependent upon the limits set by consumer acceptance of the product.

Furthermore, cultivation and marketing of transgenic products are restricted by strict regulations which strongly hamper the producer's efforts with their time-consuming and complicated bureaucracy.

Therefore, conventional breeding still remains the most suitable alternative when the characteristic that one wishes to confer on the modified wheat can be found in natural wheat populations. In fact, cross selection of the progeny can be performed depending on the presence of the desired phenotype, using protocols well known in the field.

European patent EP 0 154 039 (National Starch and Chemical Corporation) regards improvements concerning production of retorted canned pasta-based food products. In particular, the patent describes an improved canned food product, prepared starting from a pasta-based composition comprising farinaceous material and water, wherein the improvement comprises the presence in the composition of 10-40% (by weight) of high amylose corn starch (minimum 50%).

However, use of corn starch for producing pasta is not allowed by the Italian law which, according to the legislation in force (law 580/67 and subsequent amendments by Presidential Decree n°117, art. 6 of February 9, 2001), allows the denomination of durum wheat semolina pasta only for those products obtained by the exclusive use of durum wheat semolina. Furthermore, the increase in the amylose content described is not directly available from the raw material used, but it is obtained following a technological enrichment treatment.

Thus, the technical problem underlying the present invention is that of providing durum wheat grain or meal containing starch having increased amylose content, compared to grains and meal usually obtained from durum wheat, without resorting to the use of genetic engineering techniques or enrichment through technological processes.

### Summary of the invention

Such problem has been solved, according to the invention, by a grain of durum wheat selected for the characteristic of not being capable of expressing the *Sgp-1* gene, containing a durum wheat starch having an apparent amylose content of about 40% or more, by weight on the weight of the starch.

More preferably, such wheat contains starch with apparent amylose content comprised between 40.0 and 43.6% by weight on the weight of the starch.

Preferably, such wheat is tetraploid and it is not capable of expressing the *Sgp-A1* and *Sgp-B1* forms of the *Sgp-1* gene.

Preferably, such tetraploid wheat is obtained by crossing a wheat variety of the *Triticum turgidum* var. *durum* Desf. species with natural wheat mutants not capable of expressing the *Sgp-A1* or *Sgp-B1* gene. Plants derived from this crossing are thus crossed again through further crosses with durum wheat varieties, to select, at the end of this process, those durum wheat plants that are capable of producing starch having an apparent amylose content of about 40% or more, preferably of between 40.0 and 43.6%, by weight on the weight of the starch.

In other words, preferably, such tetraploid wheat is obtained by crossing a wheat variety of the *Triticum turgidum* var. *durum* Desf. species with wheat selected for the characteristic of not being capable of expressing the *Sgp-A1* or *Sgp-B1* gene, performing further crosses between the wheat plants thus obtained with durum wheat varieties, and selecting the obtained durum wheat containing starch having an apparent amylose content of about 40% or more, preferably of between 40.0 and 43.6%, by weight on the weight of the starch.

Such problem is also solved, according to the invention, by a durum wheat meal obtained from the endosperm of caryopses of durum wheat, selected for the characteristic of not being capable of expressing the *Sgp-1* gene, containing a durum wheat starch having an apparent amylose content of about 40% or more, by weight on the weight of the starch.

More preferably, such wheat contains a starch having an apparent amylose content comprised between 40.0 and 43.6% by weight on the weight of the starch.

Preferably such wheat is tetraploid and it is not capable of expressing the *Sgp-A1* and *Sgp-B1* forms of the *Sgp-1* gene.

Preferably, such tetraploid wheat is obtained by crossing a variety of wheat of the *Triticum turgidum* var. *durum* Desf. species with natural wheat mutants that are not capable of expressing the *Sgp-A1* or *Sgp-B1* gene. The plants derived from this crossing are then crossed again through further crosses with varieties of durum wheat, to select, at the end of this process, those durum wheat plants that are capable of producing starch having an apparent amylose content of about 40% or more, preferably of between 40.0 and 43.6%, by weight on the weight of the starch.

In other words, preferably, such tetraploid wheat is obtained by crossing a wheat variety of the *Triticum turgidum* var. *durum* Desf. species with wheat selected for the characteristic of not being capable of expressing the *Sgp-A1* or *Sgp-B1* gene, performing further crosses between the wheat plants thus obtained with durum wheat varieties, and selecting the obtained wheat containing starch having an apparent amylose content of about 40% or more, preferably of between 40.0 and 43.6%, by weight on the weight of the starch.

Furthermore, the present invention refers to a durum wheat starch obtained from the endosperm of caryopses of durum wheat, selected for the characteristic of not being capable of expressing the *Sgp-1* gene, having an apparent amylose content of about 40% or more, by weight on the weight of the starch.

Preferably, the apparent amylose content of such starch is comprised between 40.0 and 43.6%, by weight on the weight of the starch.

Preferably, such starch is derived from a tetraploid wheat not capable of expressing the *Sgp-A1* and *Sgp-B1* forms of the *Sgp-1* gene.

Preferably, such tetraploid wheat is obtained by crossing a variety of wheat of the *Triticum turgidum var. durum Desf.* species with wheat selected for the characteristic of not being capable of expressing the *Sgp-A1* or *Sgp-B1* gene. The plants derived from this crossing are then crossed again through further crosses with varieties of durum wheat, to select, at the end of this process, those durum wheat plants that are capable of producing starch having an apparent amylose content of about 40% or more, preferably of between 40.0 and 43.6%, by weight on the weight of the starch.

In other words, preferably, such tetraploid wheat is obtained by crossing a wheat variety of the *Triticum turgidum* var. *durum* Desf. species with wheat selected for the characteristic of not being capable of expressing the *Sgp-A1* or *Sgp-B1* gene, performing further crosses between the wheat plants thus obtained with durum wheat varieties, and selecting the obtained wheat containing starch having an apparent amylose content of about 40% or more, preferably of between 40.0 and 43.6%, by weight on the weight of the starch.

Furthermore, the present invention refers to the use of the grain, meal and/or starch of the present invention in the production of food products, in particular pastas, bread, biscuits and cakes, having prebiotic effect and a reduced glycemic index.

Finally, the present invention comprises food products obtained from the grain and/or meal and/or starch of the invention, such as pastas, bread, biscuits and cakes, having prebiotic effect and a low glycemic index, in particular pasta having prebiotic effect and a reduced glycemic index obtained from the durum wheat meal of the present invention.

### Brief description of the figures

Figure 1 shows the viscosity curves with respect to time of various genotypes of wheat, generated from an RVA *(Rapid Viscosity Analyzer)* analysis. The tested genotypes comprise: cultivar Svevo, *Sgp-A1-, Sgp-B1-, Sgp-A1-B1-, Waxy.*
Figure 2 shows the differential scanning calorimetry (DSC) analysis diagram of a traditional wheat meal sample and of a sample of the meal of the invention for characterising the meal of the present invention.
Figure 3 shows the results of the characterisation of the pasta obtained starting from the meal of the present invention and of a traditional pasta. Figure 3a shows the pasta thickness values (mm) observed following the cooking times, in boiling water, of 6, 7 and 8 minutes. Figure 3b shows the total work values (J) following the cooking times, in boiling water, of 6, 7 of 8 minutes. Figure 3c shows the rupture strength values (N) following the cooking times, in boiling water, of 6, 7 and 8 minutes.

### Detailed description of the invention

Generally, wheat starch does not exhibit great variations in the amylose to amylopectin ratio. However, some wheat genotypes have, due to natural mutations, variable relative quantities of amylose and amylopectin. Varieties with high amylose content are referred to as "high amylose" while those with high amylopectin content are referred to as "waxy".

Some wheat species are diploids, i.e. they have two copies of each chromosome, but many of them are polyploid, having four (tetraploids) or six (hexaploids) copies of each chromosome. *Triticum aestivum* L. (common wheat) is a hexaploid while *Triticum turgidum* var. *durum* Desf. (durum wheat) is a tetraploid.

Studies on wheat have shown that some enzymes responsible for starch synthesis are strongly linked to the starch granules *(starch granule protein,* SGP). Wheat has three types of SGP, called SGP-1, SGP-2 and SGP-3.

Hexaploid wheat has three SGP-1 isozymes (SGP-A1, -B1 and -D1) while tetraploid wheat has two (SGP-A1 and SGP-B1).

These proteins are associated to the synthesis of amylopectin. Therefore, modifications aimed at eliminating the expression of the *Spg-*1 gene which codifies for these proteins, lead to the development of wheat containing starch having a reduced amylopectin content and, as a consequence, having a higher apparent amylose content.

### Definitions:

The term grain is herein used to indicate a grain of wheat as such. It may be used for food purposes after application of dedicated processes such as for example cleaning, decortication, precooking, puffing, etc., upon which the grain maintains its original shape, or flaking, crushing, cooking, etc., through which the grain undergoes a transformation with respect to its original shape.

"SPG-1" belongs to the group of proteins *(starch granule protein,* SGP) associated to the starch granule of the wheat caryopsis endosperm. These proteins are characterised in that they are not washed off from the starch granule after washing in a buffer solution containing the surfactant sodium dodecyl sulfate *(SDS).*

Tetraploid wheat has an AABB genome organisation and two isozymes of SGP-1 (SGP-A1 and SGP-B1). These isozymes can be detected and identified by SDS-gel electrophoresis *(SDS-PAGE)* as distinct protein bands of 115 and 100 kDa, respectively.

Hereinafter, the terms "SGP-1", "SGP-A1" and "SGP-B1" shall be used to indicate proteins, and *"Sgp-1", "Sgp-A1"* and *"Sgp-B1"* shall be used to indicate the genes coding for the proteins SGP-1, SGP-A1 and SGP-B 1, respectively.

The expression "not capable of expressing the gene" as used herein, means that one of the SGP-1 proteins is not expressed at levels detectable by SDS-gel electrophoresis. The terms "SGP-A1-", "SGP-B1-" and *"Spg-A1-", "Spg-B1-"* are used to indicate that the protein and the gene in question, respectively, are not expressed.

The expression "selected for the characteristic", is used in this context to indicate wheat which is the result of selection within the populations of plants derived from crossing.

The expression "apparent amylose content" as used herein refers to an apparent amylose content measured through the Megazyme K-Amyl kit, which involves a selective precipitation of amylose, followed by enzymatic digestion and peroxidation reaction of the D-glucose formed, forming a compound measurable by calorimetric techniques, according to the method described in Example 1.

In the present application, the expression "of about 40% or more", for example, means that the wheat has an apparent amylose content of about 40% or more measured by weight on the weight of the starch, as described in Example 1.

The term "durum wheat" refers to a plant of the *Triticum turgidum* var. *durum* Desf. species. It is characterised in that it is a tetraploid wheat with a genome organisation of the AABB type comprising 28 chromosomes.

The term *"pasting",* in the present context, refers to the formation of a "paste" through mixing starch in water with stirring.

The expression "complete nulls" or "double nulls" refers to genotypes in which neither SGP-A1 nor SGP-B1 are expressed.

The expression "single nulls" or "single null" refers to genotypes in which SGP-A1 or SGP-B1 is not expressed.

The expression "segregating genotypes" refers to genotypes obtained after at least one crossing between the initial variety and a natural mutant or its derivative, which does not express SGP-A1 or SGP-B1.

The expression "double mutant" refers to a mutant genotype in which both *Sgp-A1* and *Sgp-B1* genes are not expressed, and thus to a mutant capable of containing the maximum relative amount of amylose.

High amylose wheat grain of the present invention may be suitably prepared starting from caryopses of wheat selected for the characteristic of not being capable of expressing *Sgp-1* according to any one of the methods known to the field.

High amylose wheat meal of the present invention may be prepared by grinding the endosperm of wheat selected for the characteristic of not being capable of expressing *Sgp-1* according to any one of the methods known to the field.

High amylose wheat starch of the present invention may be prepared by isolating starch from the endosperm of wheat selected for the characteristic of not being capable of expressing *Sgp-1* according to any one of the methods known to the field.

The present invention shall be further described with reference to some embodiment provided hereinafter for illustrative and non-limiting purposes.

### EXAMPLE 1 - Production of single null and double null wheat genotypes and determination of the amylose content in null lines for the SGP-1 proteins of durum wheat.

The new durum wheat of the invention is obtained through interspecific crossing and back-crossing techniques and traditional breeding starting from the Svevo variety of *Triticum turgidum* var. *durum* Desf..

Natural common wheat mutants each lacking an SGP-1 isoform were crossed with the Svevo variety of durum wheat. The plants obtained from these crosses were further crossed with the Svevo variety to obtain durum wheat genotypes null for one of the two SGP-1 proteins (single nulls) as similar as possible to Svevo.

Subsequently, the genotypes null for one of the two SGP-1 proteins (single nulls) were crossed with each other to obtain a complete set of combinations (two single nulls and one double null).

The segregating genotypes were then classified as heterozygotes or homozygotes through the Polymerase Chain Reaction (PCR) technique using specific primers for the *Sgp-1* regions. Such analysis allowed the identification of the *Sgp-1* double mutant homozygote durum wheat, which was then subjected to further reproduction.

The entire set of "total" and "partial null" lines for the SGP-1 in durum wheat was multiplied for the subsequent analyses of the starch characteristics.

The apparent amylose content of each combination of the "partial nulls" lines was analysed using the "Megazyme" kit according to the method provided.

The apparent amylose content of the complete set of *Sgp-1* mutants of durum wheat is given in Table 1.

**Table 1 Apparent amylose content in the set of Sgp-1 mutants of durum wheat**

| Genotypes | Sgp-A1- | Sgp-B1- | Sgp-A1-B1- | Svevo |
|---|---|---|---|---|
| Amylose Content (%) | 24.3 | 29.1 | 43.6 | 23.0 |

The amylose content in the Svevo parental genotype is of about 23%, while in the complete mutant genotype for *Sgp-1* an amylose percentage of 43.6% was observed with an increase of 89%. Such value is higher than that observed by Yamamori et *al.* (2000) on the complete common wheat *Sgp-1* mutant. The amylose content of the 2 single null genotypes, *Sgp-A1-* and *Sgp-B1-,* is, respectively, of 24.3% and 29.1%. This suggests that the *Sgp-B1* homoallele has greater impact on the amylopectin synthesis compared to the *Sgp-A1* gene, given that its absence generates an increase in the amylose content of 6.1 percentage points (+ 26.52%) compared to the Svevo genotype.

As already reported by Yamamori et *al.* (2000) with regard to common wheat, the amylose content increase, observed in the complete null genotypes, could only partially be due to the absence of *Sgp-1* proteins. The simultaneous reduction in the quantity of *Sgp-2* and *Sgp-3* proteins, observed in the null genotypes, may contribute to the increase in the relative amylose content, following the reduction in the amylopectin content.

### EXAMPLE 2 - Property of meal of the null lines for SGP-1 proteins of durum wheat

A method comprising the use of the *Rapid Visco Analyzer (RVA),* an instrument connected to a computer, which allows the determination of the viscosity properties of starch after cooking, constantly measuring the apparent viscosity, at varying shear force conditions and temperature, was used for analysing the starch pasting properties.

The RVA analyses were performed on 3-4 g of starch isolated from wheat through conventional methods and dispersed in water, using a solids percentage of 10%.

The temperature profile used involved a 1 minute and 50 second rest at 50°C, a temperature increase up to 95°C in a period of time of 6 minutes, a thermal rest at 95°C for 4 minutes and a cooling step down to 50°C in 4 minutes.

The speed of rotation of the blades was of 160 revolutions per minute (rpm) for the entire duration of the test.

The object of such method is the evaluation of the starch pasting properties without accounting for the impact on its viscoelastic behaviour of the other components present in the meal.

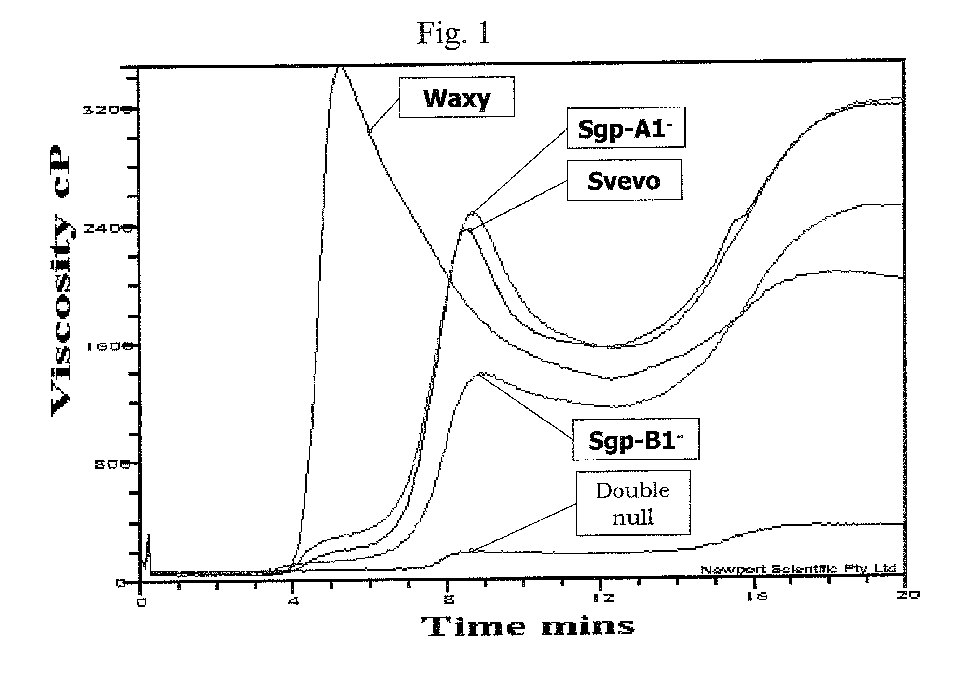

The RVA analyses performed on the "partial null" and "complete null" *Sgp-1* lines confirm the different effect of the two alleles, *Sgp-A1* and *Sgp-B1,* on the pasting properties. The meals of the complete durum wheat sample *Sgp-1* show the viscosity characteristics of the starch present therein to be very similar to those observed by Yamamori in US 7,001,939 for the "complete null" *Sgp-1* mutant of common wheat, with a very low viscosity peak value (196), *breakdown (24), setback* (181) and final viscosity (353) compared to the control (Svevo). See Figure 1.

*Viscosity peak* is defined as the maximum viscosity value measured.

*Breakdown* is described as a measure of the degree of disintegration of the starch granules or of the stability of the paste and it is calculated as the difference in viscosity between the viscosity peak value and the subsequent minimum value.

*Setback* indicates the viscosity increase due to the rearrangement of the amylose released from the starch granules during the swelling step and it is calculated as the difference between the final viscosity value and the minimum viscosity value reached by the curve after the peak.

The properties of the "single null" mutants are different: the "single null" *Sgp-A1* shows characteristics similar to Svevo, differing just slightly in the *breakdown* and in the final viscosity; on the contrary, the "single null" *Sgp-B1* line shows a lower value compared to the "single null" *Sgp-A1* line for all the parameters observed (Table 2).

**Table 2 RVA analysis on the set of partial and full Sgp-1 lines of Svevo durum wheat.**

| Sample | Visc Peak | Breakdown | Final Viscosity | Setback | Peak Time |
|---|---|---|---|---|---|
| Svevo | 2376 | 601 | 3172 | 1397 | 8.533 |
| *Sgp-A1-* | 2538 | 940 | 3305 | 1707 | 8.799 |
| *Sgp-B1-* | 1387 | 246 | 2559 | 1418 | 8.799 |
| *Sgp-A1-B1-* | 196 | 24 | 353 | 181 | 8.333 |
| *Waxy* | 3481 | 2146 | 2019 | 684 | 5.266 |

The double null mutant starch, having high amylose content and altered granules and amylopectin structure, is not capable of absorbing water during heating; as a consequence, the granules do not swell and the viscosity curve is very distinctive: in fact there are no significant viscosity increases during the RVA analyses.

The starch of the "single null" *Sgp-B1* mutant has a viscosity curve with characteristics differing to a greater extent from Svevo than the "single null" Sgp-A1 mutant starch; this supports the hypothesis that the *Sgp-B1* gene plays a greater role than the other homoallele in the synthesis of amylopectin. The RVA analyses data perfectly match those performed on the amylose content and it is interesting to observe that there is an inversely proportional correspondence between the viscosity curve and the amylose content (also see Table 3).

### EXAMPLE 3 - Standardisation of the high amylose durum wheat lines to be used in bread-making and pasta-making technological tests.

A Svevo cultivar durum wheat line with "double null" *Sgp-A1⁻ and Sgp-B1-* genotype was characterised for the starch apparent amylose content which amounted to 43.6 % of the total starch.

The meals containing high amylose starch deriving from such line are suitable for use in technological tests aimed at testing the impact of various amylose concentrations on the finished product (pasta or bread).

For producing commercial dry pasta, 600 g of high amylose semolina were mixed with 600 g of industrial semolina (having granule size in the range of 150 to 500 µm). After an initial mixing step, performed directly in the dough mixer cavity, an amount of water equivalent to 27-33% by weight with respect to the semolina to be used for pasta-making was added as two different aliquots. The mixing lasted for a period of time in the range of 15 - 30 minutes, generally for 25 minutes, at a temperature in the range of 30 - 45°C, generally at 35 °C. During the last mixing step (10-20 minutes, generally 15 minutes) a depression of 0.6-0.8 bar, generally 0.7 bar was applied. Subsequently, extrusion was performed at a pressure of 70-120 bar, generally 90 bar. The pasta produced (spaghetti) was placed on special bars and conveyed to the drying step, which was performed at a temperature in the range of 60 to 85°C, generally 70 °C, at an environmental humidity in the range of 50 to 85%, generally 60%, to bring the product humidity to 12%.

### EXAMPLE 4 - Characterisation of the high amylose meal

Meal samples obtained according to conventional methods starting from the wheat containing high amylose starch of example 3 were characterised.

In order to determine the resistant starch content, the analyses were performed through an in *vitro* enzymatic test (AOAC 2002.02). Results are summarised in Tables 3 and 4.

**Table 3 - Analytic composition of meal samples (5% coefficient of variation (CV) of data, DM = dry matter).**

| composition (% DM.) | Sample 1 (high amylose meal) | Sample 2 (standard meal) |
|---|---|---|
| Proteins | 20.7 | 12.8 |
| Lipids | 1.7 | 1.7 |
| Total carbohydrates | 68.8 | 81.2 |
| TDF (fibre) | 7.3 | 3.4 |
| Ash | 1.5 | 0.9 |

**Table 4 - Determination of the resistant starch content of the standard and high amylose meal (5% CV of the data, DM = dry matter).**

| Sample | Resistant starch (% DM) |
|---|---|
| High amylose meal | 3.5 |
| Standard meal | 0.2 |

The natural content of resistant starch (RS) in high amylose meal is 3% approximately, 15 times greater than that of standard meal (RS 0.2%). The high stability of the high amylose starch and its resistance to degradation under the action of the enzymatic pool of the organism, is due to the structural organisation of the granules themselves and/or the presence of a high percentage of amylose complexed with lipids.

### EXAMPLE 5 - Characterisation of high amylose pasta.

Samples of pasta produced starting from the meal of example 3 were characterised for their resistant starch content. The results are summarised in Table 5.

The sample referred to as "high amylose spaghetti" is represented by spaghetti, 50% made of standard meal and 50% of the meal containing the high amylose starch of example 3.

The sample referred to as "commercial pasta" is represented by conventional pasta (spaghetti) 100% made of standard durum wheat meal.

**Table 5 - Determination of the resistant starch content of pasta obtained from standard meal and having a high amylose content (5% CV of the data, DM = dry matter).**

| Sample | Resistant starch (% DM.) |
|---|---|
| High amylose spaghetti | 1.94 |
| Commercial pasta | 0.30 |

Mechanical (mixing and extrusion) and thermal *(High Temperature Drying)* treatments of the high amylose meal used in pasta-making do not alter its RS content. There is only a slight increase in RS in the pasta, compared to the meal probably due to the gelatinization and subsequent retrogradation of a part of non-resistant starch in the first high temperature drying steps. In fact, the value of 1.94 shown in Table 5, which refers to a composition containing 50% high amylose wheat, is consistent with the corresponding value in Table 4, which refers to a composition exclusively obtained from high amylose wheat.

Starch retrogradation is a phenomenon that occurs during the drying of starch previously exposed to water or humidity. Retrogradation is substantially the inverse of gelatinization and it consists in the formation of a rigid structure due to the amylose chains coming closer to each other and due to the reorganisation of the amylopectin.

### EXAMPLE 6 - Determination of the thermal characteristics of the durum wheat starch containing high amylose starch.

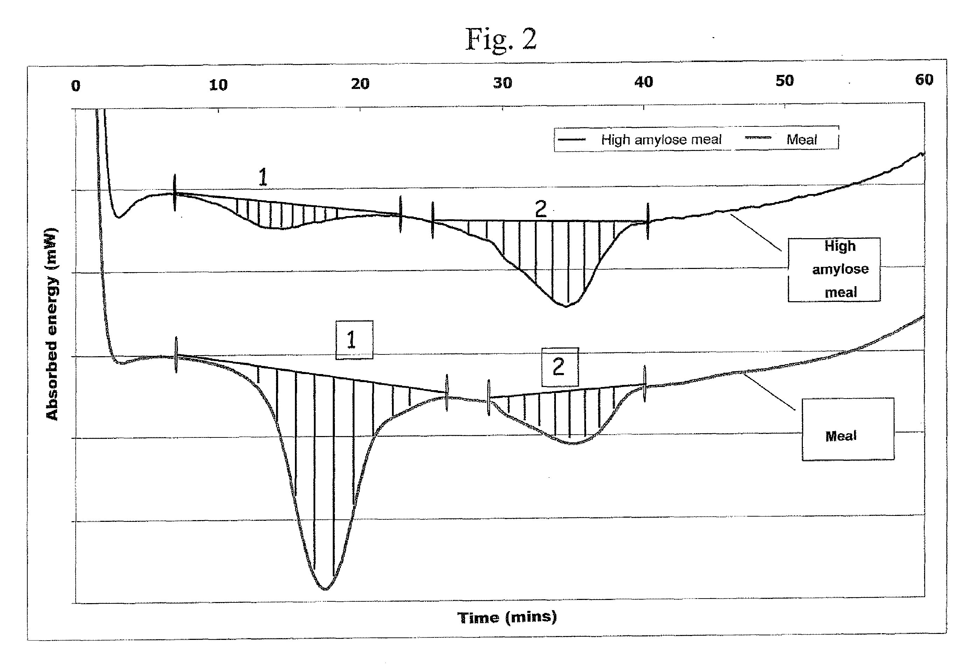

Thermodynamic parameters regarding the starch gelatinization process (swelling of granules due to water absorption and consequent loss of the crystalline structure) for the high amylose wheat meal of example 3 were measured by using differential scanning calorimetry (DSC). The results were compared with those of the conventional durum wheat meal. See Figure 2

The following parameters shall be referred to hereinafter: Starting temperature (T0), Peak temperature (Tp), Final temperature (Tc), Gelatinization enthalpy (ΔH) and amylose-lipid complex dissociation enthalpy (ΔH LAM).

The starch (3-4 mg) of the wheat meal to which water was added at a 1:2 (w/w) ratio was brought from an initial temperature of 20°C to the final temperature of 150°C in about 30 minutes.

The calorimetry analyses were performed in triplicate on the previously isolated starch samples.

The results are illustrated in Figure 2. Table 6 summarises such results.

Peak 1 represents the gelatinization while peak 2 represents the fusion of the amylose-lipid complexes.

**Table 6 - Thermal characteristics of the durum wheat starch containing high amylose starch compared to standard meal starch.**

| Sample | Peak 1 | | Peak 2 | |
|---|---|---|---|---|
| | T (°C) | ΔH | T (°C) | ΔH |
| Standard meal | 59.67 | 6.42 | 95.23 | 1.75 |
| High amylose meal | 51.34 | 1.20 | 93.7 | 3.38 |

Regarding peak 1, the starch of the meal containing high amylose starch has a Gelatinization temperature and a transition enthalpy (ΔH) lower than those of the standard meal. These observed differences could be due to a lower amount and/or degree of crystallinity of the amylopectin in the wheat granules containing high amylose starch.

The peak corresponding to the fusion of the amylose-lipid complexes (peak 2) in the meal containing high amylose starch has a considerably greater enthalpy value compared to that of the standard meal. This result indicates the presence of a higher amount of amylose and of amylose-lipid complexes.

### EXAMPLE 7 - Analysis of the texture of pasta obtained from standard meal and high amylose meal.

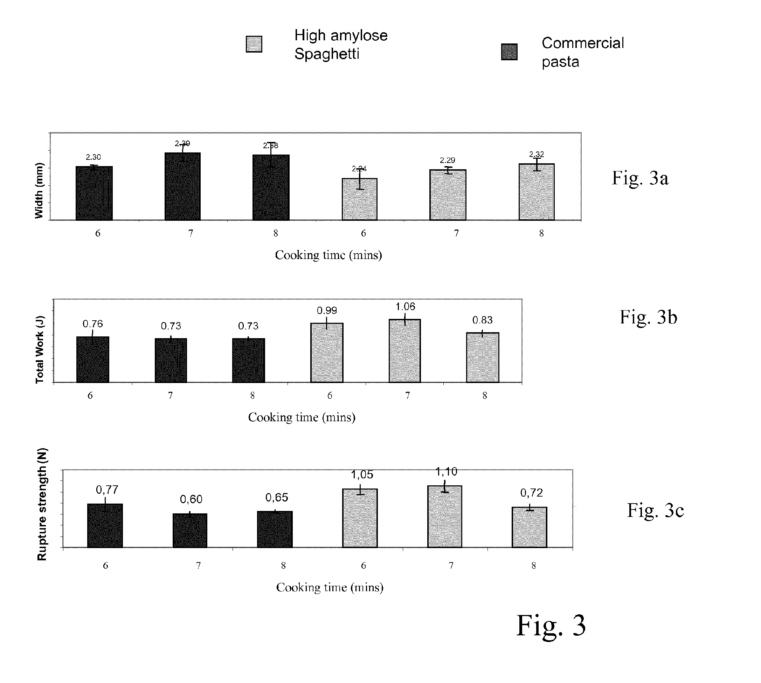

The high amylose pasta of examples 5 and 6 was then subjected to further tests aimed at analysing the texture upon cooking. The results are summarised in Figures 3a-3c.

100g of long pasta (such as spaghetti) were weighed and cooked in boiling water for a variable period of time depending on the test. At the end, the excess water was drained off and cooking was stopped by submerging the sample in cold water for 15 seconds.

The texture was measured by means of the *Texture Analyzer (Stable Micro Systems -* SMS) according to the following method.

The cooked and cooled sample was spread in a single layer on the dedicated surface of the instrument and subjected to a shear force by using a wedge having, at its lower end, a 0.5 mm thick blade proceeding towards the sample being analysed at a constant speed of 0.1 mm/second.

The arm supporting the wedge can measure the resistance to deformation and to shear offered by the sample being analysed. By monitoring the resistance offered in the period of analysis it is possible to obtain the information regarding the rheology of the sample, such as the elastic constant, rupture strength, residual thickness at rupture, total work and peak width, derivable from the curve obtained.

*Rupture strength* is described as the maximum peak height and it expresses the degree of resistance offered by the sample;

*Total work* is indicated by the area under the curve and it is independent from the shape of the curve itself.

The tests were repeated ten times.

The shear resistance tests showed a greater consistency of the spaghetti obtained from high amylose meal compared to those of the standard meals, the cooking time being equal (although the thickness of the high amylose spaghetti during cooking is always lower compared to that of standard spaghetti).

The texture difference detected between the two spaghetti samples may be due to lower water absorption kinetics by the meal containing high amylose starch, compared to the standard meal, or to an intrinsically more resistant structure of the pasta obtained from meal containing high amylose starch.

Thus, the present invention allows the provision of a durum wheat meal containing starch having an apparent amylose content of about 40% or more, preferably comprised between 40.0 and 43.6% by weight on the weight of the starch, through a method which is inexpensive and effective and which does not expose the end product to ethical and food safety concerns on the part of the average consumer.

Furthermore, the present invention refers to food products, in particular pasta obtained starting from the meal containing high amylose starch of the invention. In fact, such meal allows the production of food products having ideal organpoleptic characteristics, due to the specific amylose to amylopectin ratio of the starch.

In particular, the wheat meal of the invention may be used for the production of high amylose pasta more "al dente" or having a greater firmness, upon cooking in boiling water.

Another advantage of the food products, in particular pasta, of the present invention lies in their nutritional characteristics. In fact, the improved amylose to amylopectin ratio starch leads to an increase in the resistant starch content, compared to the starch contained in the same products made with standard meal and, consequently, to a reduction in the glycemic index of the starch and of the products obtained therefrom.

The presence of a high resistant starch content is commonly associated to a prebiotic effect at the intestinal level, with considerable health-promoting effects for the organism. The prebiotic effect consists in the modulation of the intestinal microflora by means of certain non-digestible carbohydrates, among which the resistant starch of wheat. Prebiotic intake favors the proliferation of health-promoting intestinal bacteria, such as bifidobacterium and lactobacilli, to the detriment of harmful bacteria.

The proliferation of health-promoting bacteria, is in turn associated to healthy effects, such as the cholesterol-lowering effect, the increase in mineral bioavailability, the reduction in the risk of developing colon cancer and improved lactose tolerance.

Thus, consumption of the pasta of the invention may be associated to a reduced risk of chronic diseases such as coronary heart diseases and certain types of cancer.

### Bibliographical references:

AOAC 2002.02. Measurement of Resistant Starch in Starch Samples and Plant Materials, Study director B.V. McCleary, Megazyme International Ireland Ltd., 32-40
Ball, S.G., Van de Wal, M.H.B.J., Visser, R.G.F. (1998). Progress in understanding the biosynthesis of amylose. Trends in Plant Science, 3, 462-467.
Coultate T.P. (2001) Food. The chemistry of its components, (III edition) Royal Society of Chemistry, Cambridge, U.K.
Foster-Powell K., Holt S., and Brand-Miller J. (2002) International table of glycemic index and glycemic load values. The American Journal of Clinical Nutrition, 76, 5-56.
Hu P., Zhaoa H., Duana Z., Linlina Z., Wu D. (2004) Starch digestibility and the estimated glycemic score of different types of rice differing in amylose contents. Journal of Cereal Science, 40, 231-237.
Iturriaga L.B., Lopez de Mishima B., Anon M.C. (2006) Effect of amylose on starch pastes viscoelasticity and cooked grains stickiness in rice from seven argentine genotypes. Food Research International, 39, 660-666.
Jones H.D. (2005) Wheat transformation: current technology and applications to grain development and composition. Journal of Cereal Science, 41, 137-147.
King R.A., Noakes M., Bird A.R., Morell M.K., Topping D.L. (2008) An extruded breakfast cereal made from a high amylose barley cultivar has a low glycemic index and lower plasma insulin response than one made from a standard barley. Journal of Cereal Science, 48, 526-530.
Morell, M.K., Rahman, S., Abrahams, S.L. and Appels, R. (1995) The biochemistry and molecular biology of starch synthesis in cereals. Australian Journal of Plant Physiology, 22, 647-660.Rahman S., Li Z., Batey I., Cochrane M.P., Appels R. and Morell M. (2000) Genetic Alteration of Starch Functionality in Wheat. Journal of Cereal Science, 31,91-110.
Raphaelides S.N., Georgiadis N. (2008) Effect of fatty acids on the rheological behaviour of amylomaize starch dispersions during heating. Food Research International, 41, 75-88.
Regina A., Bird A., Topping D., Bowden S., Freeman J., Barsby T., Kosar-Hashemi B., Li Z., Rahman S., and Morell M. (2006) High-amylose wheat generated by RNA interference improves indices of large-bowel health in rats. PNAS, 113 3546-3551.
Sharma R., Sissons M.J., Rathjen A. J and Jenner C. F. (2002) The Null-4A Allele at the Waxy Locus in Durum Wheat affects Pasta Cooking Quality. Journal of Cereal Science, 35, 287-297.
Topping D. (2007) Cereal complex carbohydrates and their contribution to human health. Journal of Cereal Science, 46, 220-229.
Yamamori, M., Fujita, S., Hayakawa, K., Matsuki, J. And Yasui, T. (2000). Genetic elimination of a starch granule protein, SGP-1, of wheat generates an altered starch with apparent high amylose. Theor. Appl. Genet. 101, 21-29.
Yasui T., Matsuki J., Sasaki T. and Yamamori M. (1996) Amylose and Lipid Contents, Amylopectin Structure, and Gelatinisation Properties of Waxy Wheat (Triticum aestivum) Starch. Journal of Cereal Sciences, 24, 131-137.

## Claims

1. Grain of tetraploid dump wheat suitable for the production of food products, selected for the characteristic of not expressing the *Sgp-A1* and *Sgp-B1* forms of the *Sgp-1* gene, containing a durum wheat starch having an apparent amylose content of about 40% or more by weight on the weight of the starch.

2. Grain of tetraploid durum wheat according to claim 1 wherein said tetraploid wheat is obtained by crossing a wheat variety of the *Triticum turgidum* var. *durum* Desf. species with wheat selected for the characteristic of not expressing the *Sgp-A1* or *Sgp-B1* gene, performing further crosses between the wheat plants thus obtained with durum wheat varieties, and selecting the obtained durum wheat containing starch having an apparent amylose content of about 40% or more by weight on the weight of the starch.

3. Grain according to claim 1 or 2 wherein said durum wheat starch has an apparent amylose content comprised between 40.0 and 43.6% by weight on the weight of the starch.

4. Durum wheat meal obtained from the endosperm of caryopses of tetraploid durum wheat suitable for the production of food products, selected for the characteristic of not expressing the *Sgp-A1* and *Sgp-B1* forms of the *Sgp-1* gene, containing a durum wheat starch having an apparent amylose content of about 40% or more by weight on the weight of the starch.

5. Meal according to claim 4 wherein said tetraploid wheat is obtained by crossing a wheat variety of the *Triticum turgidum* var. *durum* Desf. species with wheat selected for the characteristic of not expressing the *Sgp-A1* or *Sgp-B1* gene, performing further crosses between the wheat plants thus obtained with durum wheat varieties, and selecting the obtained wheat containing starch having an apparent amylose content of about 40% or more by weight on the weight of the starch.

6. Meal according to claim 4 or 5 wherein said durum wheat starch has an apparent amylose content comprised between 40.0 and 43.6% by weight on the weight of the starch.

7. Durum wheat starch obtained from the endosperm of a caryopsis of tetraploid durum wheat suitable for the production of food products, selected for the characteristic of not expressing the *Sgp-A1* and *Sgp-B1* formes of the *Sgp-1* gene, having an apparent amylose content of about 40% or more by weight on the weight of the starch.

8. Starch according to claim 7 wherein said tetraploid wheat is obtained by crossing a wheat variety of the *Triticum turgidum* var. *durum* Desf. species with wheat selected for the characteristic of not expressing the *Sgp-A1* or *Sgp-B1* gene, performing further crosses between the wheat plants thus obtained with durum wheat varieties, and selecting the obtained wheat containing starch having an apparent amylose content of about 40% or more by weight on the weight of the starch.

9. Starch according to claim 7 or 8 wherein said apparent amylose content of said starch is comprised between 40.0 and 43.6% by weight on the weight of the starch.

10. Use of grain of durum wheat according to any one of claims 1 to 3 for the production of pastas, bread, biscuits and cakes, having prebiotic effect and a reduced glycemic index.

11. Use of meal according to any one of claims 4 to 6 for the production of pastas, bread, biscuits and cakes, having prebiotic effect and a reduced glycemic index.

12. Use of high amylose starch according to any one of claims 7 to 9 for the production of pastas, bread, biscuits and cakes, having prebiotic effect and a reduced glycemic index.

13. Food products having prebiotic effect and a reduced glycemic index obtained from grain, meal and/or starch according to any one of claims 1 to 9 wherein such products comprise pastas, bread, biscuits and cakes.

14. Pasta having prebiotic effect and a reduced glycemic index obtained from the meal according to any one of claims 4 to 6.

## Amended claims

### Amended claims in accordance with Rule 137(2) EPC.

**1.** Grain of tetraploid durum wheat suitable for the production of food products, selected for the characteristic of not expressing the *Sgp-A1* and *Sgp-B1* forms of the *Sgp-1* gene, containing a durum wheat starch having an apparent amylose content of about 40% or more by weight on the weight of the starch.

**2.** Grain of tetraploid durum wheat according to claim 1 wherein said tetraploid wheat is obtained by crossing a wheat variety of the *Triticum turgidum* var. *durum* Desf. species with wheat selected for the characteristic of not expressing the *Sgp-A1* or *Sgp-B1* gene, performing further crosses between the wheat plants thus obtained with durum wheat varieties, and selecting the obtained durum wheat containing starch having an apparent amylose content of about 40% or more by weight on the weight of the starch.

**3.** Grain according to claim 1 or 2 wherein said durum wheat starch has an apparent amylose content comprised between 40.0 and 43.6% by weight on the weight of the starch.

**4.** Durum wheat meal obtained from the endosperm of caryopses of tetraploid durum wheat suitable for the production of food products, selected for the characteristic of not expressing the *Sgp-A1* and *Sgp-B1* forms of the *Sgp-1* gene, containing a durum wheat starch having an apparent amylose content of about 40% or more by weight on the weight of the starch.

**5.** Meal according to claim 4 wherein said tetraploid wheat is obtained by crossing a wheat variety of the *Triticum turgidum* var. *durum* Desf. species with wheat selected for the characteristic of not expressing the *Sgp-A1* or *Sgp-B1* gene, performing further crosses between the wheat plants thus obtained with durum wheat varieties, and selecting the obtained wheat containing starch having an apparent amylose content of about 40% or more by weight on the weight of the starch.

**6.** Meal according to claim 4 or 5 wherein said durum wheat starch has an apparent amylose content comprised between 40.0 and 43.6% by weight on the weight of the starch.

**7.** Durum wheat starch obtained from the endosperm of a caryopsis of tetraploid durum wheat suitable for the production of food products, selected for the characteristic of not expressing the *Sgp-A1* and *Sgp-B1* forms of the *Sgp-1* gene, having an apparent amylose content of about 40% or more by weight on the weight of the starch.

**8.** Starch according to claim 7 wherein said tetraploid wheat is obtained by crossing a wheat variety of the *Triticum turgidum* var. *durum* Desf. species with wheat selected for the characteristic of not expressing the *Sgp-A1* or *Sgp-B1* gene, performing further crosses between the wheat plants thus obtained with durum wheat varieties, and selecting the obtained wheat containing starch having an apparent amylose content of about 40% or more by weight on the weight of the starch.

**9.** Starch according to claim 7 or 8 wherein said apparent amylose content of said starch is comprised between 40.0 and 43.6% by weight on the weight of the starch.

**10.** Use of grain of durum wheat according to any one of claims 1 to 3 for the production of pastas, bread, biscuits and cakes, having prebiotic effect and a reduced glycemic index.

**11.** Use of meal according to any one of claims 4 to 6 for the production of pastas, bread, biscuits and cakes, having prebiotic effect and a reduced glycemic index.

**12.** Use of high amylose starch according to any one of claims 7 to 9 for the production of pastas, bread, biscuits and cakes, having prebiotic effect and a reduced glycemic index.

**13.** Food products having prebiotic effect and a reduced glycemic index obtained from grain, meal and/or starch according to any one of claims 1 to 9 wherein such products comprise pastas, bread, biscuits and cakes.

**14.** Pasta having prebiotic effect and a reduced glycemic index obtained from the meal according to any one of claims 4 to 6.
